# EUROPÄISCHE PATENTSCHRIFT

(11) **EP 3 038 998 B1**
(45) Veröffentlichungstag und Bekanntmachung des Hinweises auf die Patenterteilung: **07.06.2017**
(21) Anmeldenummer: 14758350.4
(22) Anmeldetag: 28.08.2014
(51) Int. Cl.: C07C 2/78, B01J 4/00, B01F 5/00

(54) **VORRICHTUNG UND VERFAHREN ZUM HERSTELLEN VON ACETYLEN UND SYNTHESEGAS**
DEVICE AND METHOD FOR PRODUCING ACETYLENE AND SYNTHESIS GAS
DISPOSITIF ET PROCÉDÉ DE PRODUCTION D'ACÉTYLÈNE ET DE GAZ DE SYNTHÈSE

(30) Priorität: 29.08.2013 EP 13182228
(43) Veröffentlichungstag der Anmeldung: 06.07.2016
(73) Patentinhaber: BASF SE, 67056 Ludwigshafen am Rhein (DE)
(72) Erfinder: KERN, Matthias, 67146 Deidesheim (DE); RUSS, Michael, 67354 Römerberg (DE); RENZE, Peter, 68163 Mannheim (DE); VICARI, Maximilian, 67117 Limburgerhof (DE)
(86) Internationale Anmeldenummer: PCT/EP2014/068235
(87) Internationale Veröffentlichungsnummer: WO 2015/028540

(56) Entgegenhaltungen:
- DE-A1- 4 422 815
- DE-A1-102005 001 900
- DE-A1-102005 018 981
- DE-C- 875 198

## Beschreibung

Die vorliegende Erfindung betrifft eine verbesserte Vorrichtung und ein verbessertes Verfahren zum Herstellen von Acetylen und Synthesegas durch partielle Oxidation von Kohlenwasserstoffen in einem Reaktor, bei welchen man dem Reaktor einen den Kohlenwasserstoff enthaltenden Strom sowie einen den Sauerstoff enthaltenden Strom zuleitet.

Hochtemperaturreaktionen zur partiellen Oxidation von Kohlenwasserstoffen werden üblicherweise in einem Reaktorsystem aus Mischeinheit, Brennerblock, Feuerraum und Quencheinrichtung durchgeführt. Als Beispiel für eine solche partielle Oxidation im Hochtemperaturbereich ist die Herstellung von Acetylen und Synthesegas durch partielle Oxidation von Kohlenwasserstoffen zu nennen. Diese wird beispielsweise in der DE 875198, DE 1051845, DE 1057094 und DE 4422815 beschrieben.

Hierin werden die für das BASF-Sachsse-Bartholomé-Acetylenverfahren üblicherweise eingesetzten Mischer/Brennerblock/Feuerraum/Quench-Kombinationen - im Folgenden, wenn auf die Kombination Bezug genommen wird, vereinfacht als Reaktor bezeichnet - erläutert.

Die Ausgangsstoffe, wie beispielsweise Erdgas und Sauerstoff werden dabei getrennt aufgeheizt, üblicherweise bis zu 600°C. In einer Mischzone werden die Reaktanden intensiv vermischt und nach Durchströmen eines Brennerblocks in einem Feuerraum zur exothermen Reaktion gebracht. Der Brennerblock besteht in diesen Fällen aus einer bestimmten Anzahl aus parallelen Kanälen, in denen die Strömungsgeschwindigkeit der zündfähigen Sauerstoff/Erdgas-Mischung höher ist als die Flammengeschwindigkeit (Reaktionsgeschwindigkeit, Umsetzungsgeschwindigkeit), um ein Durchschlagen der Flamme in den Mischraum zu verhindern. Der metallische Brennerblock wird gekühlt, um den thermischen Belastungen standzuhalten. Je nach Aufenthaltszeit im Mischraum entsteht die Gefahr der Vor- und Rückzündung aufgrund der begrenzten thermischen Stabilität der Mischungen. Hier wird der Begriff der Zündungsverzugszeit bzw. Induktionszeit gebraucht als diejenige Zeitspanne, in der eine zündfähige Mischung keine nennenswerte intrinsische thermische Veränderung durchläuft. Die Induktionszeit ist abhängig von der Art der eingesetzten Kohlenwasserstoffe, dem Mischungszustand, von Druck und Temperatur. Sie bestimmt die maximale Aufenthaltszeit der Reaktanden im Mischraum. Reaktanden wie Wasserstoff, Flüssiggas oder Leichtbenzin, deren Einsatz aufgrund von Ausbeute- und/oder Kapazitätssteigerungen im Syntheseprozess besonders wünschenswert ist, zeichnen sich durch eine vergleichsweise hohe Reaktivität und damit geringe Induktionszeit aus.

Trotz der durch diese Vorrichtungen bewirkten Vorteile besteht nach wie vor ein Potenzial für Verbesserungen. So ist bei der Acetylensynthese nach Sachsse-Bartholomé eine vollständige Vermischung der Eduktströme von Bedeutung. Hierbei kann es bei den bekannten Vorrichtungen zu einem ungewollten hydrodynamischen Druckstoß kommen. Dies limitiert insbesondere bei hohen Durchsätzen die Kapazität der Anlage, da eine Durchsatzsteigerung nur noch über eine Druckerhöhung erreicht werden kann. Weiterhin kann es durch eine asymmetrische Anströmung eines mischungsoptimierenden Drallregisters zu einer Fehlverteilung der Eduktkonzentration sowie der Massenstromdichte in der Mischeinrichtung kommen, die im folgenden Reaktor zu einer Reduktion der Ausbeute führt.

Es ist daher Aufgabe der vorliegenden Erfindung, eine Vorrichtung und ein Verfahren zu geben, das die oben beschriebenen Nachteile zumindest weitgehend vermindert und insbesondere mögliche Fehlverteilungen stromabwärts des Drallregisters vermeidet.

Ein Grundgedanke der vorliegenden Erfindung ist, durch eine geeignete Auslegung des Drallregisters, welche über die sogenannte Drallzahl und die Art und Ausrichtung der Drall erzeugenden Umlenkbleche spezifiziert werden kann, eine möglichen Fehlverteilung nach dem Drallregister zu vermeiden. Durch das erfindungsgemäße Design des Drallregisters und des Querschnitts der Mischeinrichtung kann ein ungewollter hydrodynamischer Druckstoß in der Mischeinrichtung vermieden werden. Dies kann ebenfalls über die charakteristischen Kennzahlen Drallzahl sowie Reynoldszahl spezifiziert werden.

Eine erfindungsgemäße Vorrichtung zum Herstellen von Acetylen und Synthesegas durch partielle Oxidation von Kohlenwasserstoffen mit Sauerstoff umfasst eine Mischeinheit, einen Mischabschnitt und einen Mischdiffusor. Die Mischeinheit weist eine Zufuhröffnung zum Zuführen eines kohlenwasserstoffhaltigen Stroms, eine Zufuhröffnung zum Zuführen eines sauerstoffhaltigen Stroms, ein Drallregister und eine Verteilerplatte auf. Die Verteilerplatte ist zwischen dem Mischabschnitt und der Zufuhröffnung zum Zuführen eines kohlenwasserstoffhaltigen Stroms angeordnet. Die Verteilerplatte weist Öffnungen auf. Das Drallregister ist zwischen der Zufuhröffnung zum Zuführen eines sauerstoffhaltigen Stroms und dem Mischabschnitt angeordnet. Der Mischdiffusor ist mit dem Mischabschnitt verbunden.

Der Mischabschnitt kann im Wesentlichen zylindrisch ausgebildet sein. Die Verteilerplatte kann so ausgebildet sein, dass die Verteilerplatte den Mischabschnitt in einer Umfangsrichtung gesehen vollständig umgibt. Das Drallregister kann zumindest teilweise innerhalb der Verteilerplatte angeordnet sein. Die Verteilerplatte kann Öffnungen zum Verteilen des kohlenwasserstoffhaltigen Stroms aufweisen. Die Öffnungen können bevorzugt gleichmäßig verteilt in der Verteilerplatte angeordnet sein. Alternativ können die Öffnungen ungleichmäßig verteilt in der Verteilerplatte angeordnet sein. Die Verteilerplatte kann so ausgebildet sein, dass das Verhältnis der Summe der Querschnittsflächen der Öffnungen zu einer Oberfläche der Verteilerplatte von 0,1 bis 0,4, bevorzugt von 0,2 bis 0,3 und noch bevorzugter von 0,2 bis 0,25 ist, beispielsweise 0,225. Die Zufuhröffnung zum Zuführen eines kohlenwasserstoffhaltigen Stroms kann im Wesentlichen radial bezüglich einer Mittelachse des Mischabschnitts angeordnet sein. Die Zufuhröffnung zum Zuführen eines sauerstoffhaltigen Stroms kann im Wesentlichen parallel zu einer Mittelachse des Mischabschnitts angeordnet sein. Der Mischabschnitt kann mit einem Mischrohr verbunden sein, das eine Länge und einen Durchmesser aufweist, wobei die Länge und der Durchmesser so ausgebildet sind, dass eine Mischung aus dem sauerstoffhaltigen Strom und dem kohlenwasserstoffhaltigen Strom bei Betrieb der Vorrichtung zum Herstellen von Acetylen und Synthesegas durch partielle Oxidation von Kohlenwasserstoffen mit Sauerstoff in dem Mischrohr eine Strömungsgeschwindigkeit von 0,2 Mach bis 1 Mach, bevorzugt von 0,3 Mach bis 0,9 Mach und noch bevorzugter von 0,4 Mach bis 0,8 Mach aufweist, beispielsweise 0,6 Mach. Das Drallregister kann eine Drallzahl von 0,3 bis 1, bevorzugt von 0,5 bis 0,7 und noch bevorzugter von 0,55 bis 0,65 aufweisen, beispielsweise 0,6. Mit anderen Worten weist eine das Drallregister durchströmende Strömung einen Strömungszustand mit einer derartigen Drallzahl auf.

Ein erfindungsgemäßes Verfahren zum Herstellen von Acetylen und Synthesegas durch partielle Oxidation von Kohlenwasserstoffen mit Sauerstoff umfasst eine Mischeinheit, einen Mischabschnitt und einen Mischdiffusor. Ein kohlenwasserstoffhaltiger Strom wird zu dem Mischabschnitt durch eine Zufuhröffnung zugeführt. Ein sauerstoffhaltiger Strom wird zu dem Mischabschnitt durch eine Zufuhröffnung zugeführt. Eine Verteilerplatte wird zwischen dem Mischabschnitt und der Zufuhröffnung zum Zuführen eines kohlenwasserstoffhaltigen Stroms angeordnet. Die Verteilerplatte weist Öffnungen auf. Das Drallregister wird zwischen der Zufuhröffnung zum Zuführen eines sauerstoffhaltigen Stroms und dem Mischabschnitt angeordnet. Ein Mischdiffusor wird mit dem Mischabschnitt verbunden.

Die Verteilerplatte kann so ausgebildet sein, dass das Verhältnis der Summe der Querschnittsflächen der Öffnungen zu einer Oberfläche der Verteilerplatte von 0,1 bis 0,4, bevorzugt von 0,2 bis 0,3 und noch bevorzugter von 0,2 bis 0,25 ist, beispielsweise 0,225. Der kohlenwasserstoffhaltige Strom kann im Wesentlichen radial bezüglich einer Mittelachse des Mischabschnitts zugeführt werden. Der sauerstoffhaltige Strom kann im Wesentlichen parallel zu einer Mittelachse des Mischabschnitts zugeführt werden. Das Mischrohr kann eine Länge und einen Durchmesser aufweisen, wobei die Länge und der Durchmesser so ausgebildet sind, dass das Verfahren zum Herstellen von Acetylen und Synthesegas durch partielle Oxidation von Kohlenwasserstoffen mit Sauerstoff so durchgeführt wird, dass eine Mischung aus dem sauerstoffhaltigen Strom und dem kohlenwasserstoffhaltigen Strom in dem Mischrohr eine Strömungsgeschwindigkeit von 0,2 Mach bis 1 Mach, bevorzugt von 0,3 Mach bis 0,9 Mach und noch bevorzugter von 0,4 Mach bis 0,8 Mach aufweist, beispielsweise 0,6 Mach. Das Drallregister kann eine Drallzahl von 0,3 bis 1, bevorzugt von 0,5 bis 0,7 und noch bevorzugter von 0,55 bis 0,65 aufweisen, beispielsweise 0,6.

Unter einer Mischeinheit eines Reaktors ist im Rahmen der vorliegenden Erfindung derjenige Bereich des Reaktors zu verstehen, in dem die getrennt aufgeheizten Ausgangsstoffe, wie beispielsweise Erdgas und Sauerstoff, zusammengeführt werden und sich zu mischen beginnen. Der Mischvorgang findet dabei in einem Mischabschnitt statt.

Unter einem Mischrohr ist im Rahmen der vorliegenden Erfindung ein rohrförmiges Bauteil zu verstehen, in dem die getrennt aufgeheizten und bereits zusammengeführten Ausgangsstoffe intensiv vermischt werden. Da die Ausgangstoffe in dem Mischrohr eine hohe Strömungsgeschwindigkeit von 0,2 Mach bis 1 Mach aufweisen, werden diese schnell verteilt bzw. vermischt.

Unter einem Mischdiffusor ist im Rahmen der vorliegenden Erfindung ein Bauteil zu verstehen, das Gas- und/oder Flüssigkeitsströmungen verlangsamt und die Mischung und Strömungsgeschwindigkeiten homogenisiert. Im Bereich der vorliegenden Erfindung wird dies durch eine Vergrößerung des Durchflussquerschnittes in Strömungsrichtung des strömenden Mediums erreicht.

Unter einer Verteilerplatte ist im Rahmen der vorliegenden Erfindung ein plattenförmiges Bauteil zum Verteilen des kohlenwasserstoffhaltigen Stroms auf den Mischabschnitt zu verstehen. Die Verteilerplatte muss dabei nicht eben oder plan ausgebildet sein, sondern kann beispielsweise zylindrisch ausgebildet sein. Die Verteilerplatte weist Öffnungen auf. Die Öffnungen sind zum Verteilen des kohlenwasserstoffhaltigen Stroms auf den Mischabschnitt ausgebildet, indem der kohlenwasserstoffhaltige Strom durch die Öffnungen in den Mischabschnitt gelangt. Da die Öffnungen über die Platte verteilt angeordnet sind, wird der kohlenwasserstoffhaltige Strom auf den Mischabschnitt verteilt.

Unter einem Drallregister ist im Rahmen der vorliegenden Erfindung ein Bauteil zu verstehen, das dem sauerstoffhaltigen Strom einen Drall versetzt, so dass eine Drallströmung gebildet wird. Der Drall kann beispielsweise mittels Umlenkblechen erzeugt werden, die den sauerstoffhaltigen Strom bei Überströmen umlenken. Drallströmungen können neben ihrem allgemeinen Strömungszustand, wie beispielsweise laminar oder turbulent, noch spezifischer über die Drallzahl charakterisiert werden. Die Drallzahl stellt das Verhältnis aus axialem und rotativem Impuls einer Strömung dar. Dabei wird der Drehimpuls mit einem charakteristischen Längenmaß der Strömung wie beispielsweise dem Außenradius der Strömungsberandung, die von einem durchströmten Bauteil definiert wird, gewichtet. Über die Messung des Geschwindigkeitsprofils von tangentialen und axialen Geschwindigkeiten lässt sich somit die Drallzahl bestimmen. Generell gilt, je höher die Umfangskomponenten des Geschwindigkeitsfelds verglichen mit den Axialgeschwindigkeiten der Drallströmung sind, desto höher ist die Drallzahl.

Als weitere mögliche Kennzahl für den Strömungszustand wird im Rahmen der vorliegenden Erfindung die Reynolds-Zahl verwendet. Die Reynolds-Zahl ist eine dimensionslose Kennzahl. Sie stellt das Verhältnis von Trägheits- zu Zähigkeitskräften dar. Bei Rohrströmungen werden als charakteristische Größen üblicherweise der Innendurchmesser, der Betrag der über den Querschnitt gemittelten Geschwindigkeit und die Viskosität des Fluids verwendet. Das Verhältnis des Produkts aus der gemittelten Geschwindigkeit mit dem Innendurchmesser zu der Viskosität bildet die Reynolds-Zahl. Unterhalb einer kritischen Reynolds-Zahl von ungefähr 2300 ist die Strömung im Allgemeinen laminar und oberhalb davon im Allgemeinen turbulent.

Unter einem Brennerblock eines Reaktors ist im Rahmen der vorliegenden Erfindung derjenige Bereich des Reaktors zu verstehen, den die aufgeheizten und vermischten Ausgangsstoffe durchströmen. Die vermischten Ausgangsstoffe strömen dabei in Kanälen oder Bohrungen. Sofern nicht anders angegeben werden im Rahmen der vorliegenden Erfindung die Ausdrücke Kanal und Bohrung synonym verwendet. Unter einer Bohrung ist im Rahmen der vorliegenden Erfindung ein runder oder unrunder Durchbruch in einem Bauteil zu verstehen.

Unter einem Feuerraum eines Reaktors ist im Rahmen der vorliegenden Erfindung derjenige Bereich des Reaktors zu verstehen, in den die aufgeheizten und vermischten Ausgangsstoffe nach Durchströmen des Brennerblocks gelangen und zur exothermen Reaktion gebracht werden. Da im Rahmen der vorliegenden Erfindung als Ausgangstoffe Kohlenwasserstoff und Sauerstoff verwendet werden, bildet sich bei dieser exothermen Reaktion hauptsächlich Acetylen. Als weitere Produkte entstehen Wasserstoff, Kohlenmonoxid und in geringem Umfang Ruß.

Unter einer Querschnittsfläche ist im Rahmen der vorliegenden Erfindung der Flächeninhalt der bei einem Querschnitt freigelegten Fläche. Der Querschnitt verläuft dabei senkrecht zu einer Mittellinie desjenigen Bauteils, auf dessen Querschnittsfläche Bezug genommen wird. Beispielsweise ist die Querschnittsfläche einer Öffnung derjenige Flächeninhalt, der bei einem Querschnitt senkrecht zu einer gedachten Mittellinie durch den Mittelpunkt der Öffnung ermittelbar ist.

Unter einem regelmäßigen Muster ist im Rahmen der vorliegenden Erfindung ein Muster zu verstehen, das aus verschiedenen Elementen in einer vorbestimmten symmetrischen oder gleichmäßigen Ordnung besteht. Mit anderen Worten sind die Elemente in festen örtlichen Abständen zueinander wiederholt angeordnet.

Unter dem Ausdruck im Wesentlichen ist im Rahmen der vorliegenden Erfindung eine Abweichung von nicht mehr als 20 ° oder 20 % und bevorzugt nicht mehr als 15 ° oder 15 % von einer exakten Ausrichtung zu verstehen. So bedeutet beispielsweise im Wesentlichen radial bezüglich einer Mittelachse eine Abweichung von einer exakt radialen Richtung von nicht mehr als 20 ° und bevorzugt nicht mehr als 15 °. Analog bedeutet beispielsweise im Wesentlichen parallel bezüglich einer Mittelachse eine Abweichung von einer exakt parallelen Ausrichtung von nicht mehr als 20 ° und bevorzugt nicht mehr als 15 °. Bei der Angabe einer Form eines Bauteils, wie beispielsweise im Wesentlichen zylindrisch, bedeutet im Wesentlichen, dass die Form um nicht mehr als 20 % und bevorzugt nicht mehr als 15 %von der idealen Form abweicht, wobei sich die Prozentangabe auf eine Oberfläche des Bauteils bezieht.

Weitere optionale Einzelheiten und Merkmale der vorliegenden Erfindung ergeben sich aus der nachfolgenden Beschreibung bevorzugter Ausführungsbeispiele, welche schematisch in den Zeichnungen dargestellt sind.

Es zeigen:
Figur 1 eine Schnittansicht einer Vorrichtung zum Herstellen von Acetylen und Synthesegas
Figur 2eine Darstellung einer Verteilerplatte in einem gedachten aufgerollten Zustand und
Figur 3ein Simulationsergebnis der Simulation eines Verhältnisses einer lokalen Geschwindigkeit zu einer mittleren Geschwindigkeit über den Umfang des Mischrohrs.

### Ausführungsformen der Erfindung

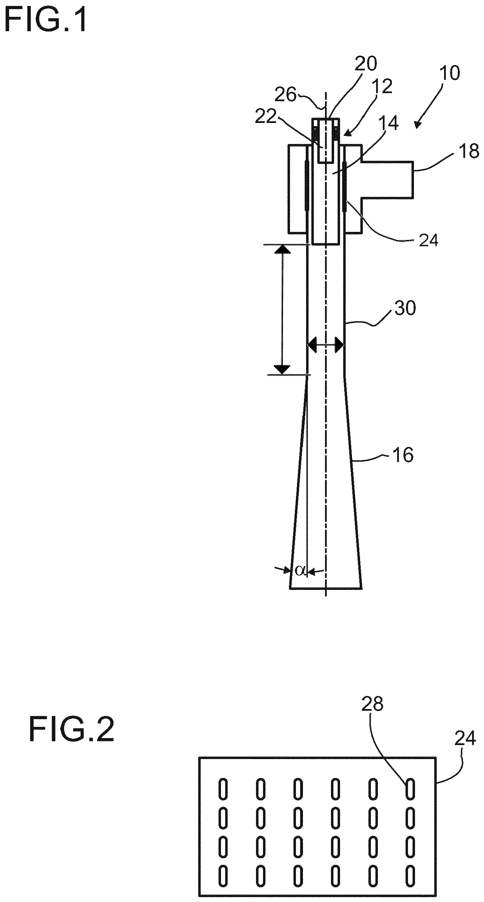

Figur 1 zeigt eine perspektivische Schnittansicht einer erfindungsgemäßen Vorrichtung zum Herstellen von Acetylen und Synthesegas durch partielle Oxidation von Kohlenwasserstoffen mit Sauerstoff. Die Vorrichtung 10 umfasst eine Mischeinheit 12, einen Mischabschnitt 14 und einen Mischdiffusor 16. Die Mischeinheit 12 weist eine Zufuhröffnung 18 zum Zuführen eines kohlenwasserstoffhaltigen Stroms, wie beispielsweise Erdgas, eine Zufuhröffnung 20 zum Zuführen eines sauerstoffhaltigen Stroms, ein Drallregister 22 und eine Verteilerplatte 24 auf. Der Mischabschnitt 14 schließt sich an das Drallregister 22 an.

Der Mischabschnitt 14 ist mit dem Mischdiffusor 16 verbunden. Der Mischabschnitt 14 ist im Wesentlichen zylindrisch ausgebildet und weist eine Mittelachse 26 auf. Die Zufuhröffnung 18 zum Zuführen eines kohlenwasserstoffhaltigen Stroms ist im Wesentlichen radial bezüglich der Mittelachse 26 des Mischabschnitts 14 angeordnet. Die Zufuhröffnung 20 zum Zuführen eines sauerstoffhaltigen Stroms ist im Wesentlichen parallel zu der Mittelachse 26 des Mischabschnitts 14 angeordnet. Die Verteilerplatte 24 ist zwischen dem Mischabschnitt 14 und der Zufuhröffnung 18 zum Zuführen eines kohlenwasserstoffhaltigen Stroms angeordnet. Die Verteilerplatte 24 umgibt den Mischabschnitt 14 in einer Umfangsrichtung um die Mittelachse 26 gesehen vollständig.

Figur 2 zeigt eine Darstellung der Verteilerplatte 24 in einem gedachten aufgerollten Zustand. Die Verteilerplatte 24 weist zum Verteilen des kohlenwasserstoffhaltigen Stroms Öffnungen 28 auf. Die Öffnungen 28 sind bevorzugt gleichmäßig verteilt in der Verteilerplatte 24 angeordnet sein. Die Öffnungen 28 sind beispielsweise durch Fräsen in die Verteilerplatte 24 eingebracht. Die Öffnungen 28 sind beispielsweise langlochförmig oder oval. Die jeweils längere Halbachse einer derartigen Form der Öffnungen 28 kann beispielsweise parallel zu einer Höhe der Verteilerplatte angeordnet sein und somit senkrecht zu der Umfangsrichtung der Verteilerplatte 24 um die Mittelachse 26. Alternativ können die Öffnungen 28 ungleichmäßig verteilt in der Verteilerplatte 24 angeordnet sein. Die Verteilerplatte 24 ist so ausgebildet, dass das Verhältnis der Summe der Querschnittsflächen der Öffnungen 28 zu einer Oberfläche der Verteilerplatte 24 von 0,1 bis 0,4, bevorzugt von 0,2 bis 0,3 und noch bevorzugter von 0,2 bis 0,25 ist, beispielsweise 0,225.

Das Drallregister 22 ist zwischen der Zufuhröffnung 20 zum Zuführen eines sauerstoffhaltigen Stroms und dem Mischabschnitt 14 angeordnet. Der Mischabschnitt 14 schließt sich somit an das Drallregister 22 an. Das Drallregister 22 ist zumindest teilweise innerhalb der Verteilerplatte 24 angeordnet. Um den sauerstoffhaltigen Strom einen Drall zu versetzen, weist das Drallregister 22 nicht näher gezeigte Umlenkbleche auf. Das Drallregister 22 weist eine Drallzahl von 0,3 bis 1, bevorzugt von 0,5 bis 0,7 und noch bevorzugter von 0,55 bis 0,65 aufweisen, beispielsweise 0,6.

Wie oben erwähnt, ist der Mischabschnitt 14 mit dem Mischdiffusor 16 verbunden. Der Mischabschnitt 14 ist genauer mit einem Mischrohr 30 verbunden, das wiederum mit dem Mischdiffusor 16 verbunden ist. Der Mischdiffusor 16 kann einen halben Öffnungswinkel α von kleiner 8 °, bevorzugt kleiner 4 ° und noch bevorzugter kleiner 2 ° aufweisen, beispielsweise 1,5 °.Das Mischrohr 30 weist eine Länge und einen Durchmesser auf. Die Länge und der Durchmesser sind dabei so ausgebildet bzw. festgelegt, dass eine Mischung aus dem sauerstoffhaltigen Strom und dem kohlenwasserstoffhaltigen Strom bei Betrieb der Vorrichtung 10 zum Herstellen von Acetylen und Synthesegas durch partielle Oxidation von Kohlenwasserstoffen mit Sauerstoff in dem Mischrohr 30 eine Strömungsgeschwindigkeit von 0,2 Mach bis 1 Mach, bevorzugt von 0,3 Mach bis 0,9 Mach und noch bevorzugter von 0,4 Mach bis 0,8 Mach aufweist, beispielsweise 0,6 Mach.

Nachstehend wird ein erfindungsgemäßes Verfahren zum Herstellen von Acetylen und Synthesegas durch partielle Oxidation von Kohlenwasserstoffen mit Sauerstoff beschrieben, das bei der Vorrichtung 10 zum Einsatz kommen kann. Die Kohlenwasserstoffe, wie beispielsweise Erdgas, und der Sauerstoff werden als Ausgangsgase getrennt vorerhitzt und als getrennte Ströme vorgesehen. Ein kohlenwasserstoffhaltiger Strom wird dem Mischabschnitt 14 durch die Zufuhröffnung 18 zugeführt. Auf Grund der besonderen oben beschriebenen Anordnung der Zuführöffnung 18, wird der kohlenwasserstoffhaltige Strom im Wesentlichen radial bezüglich der Mittelachse 26 des Mischabschnitts 14 zugeführt. Ein sauerstoffhaltiger Strom wird dem Mischabschnitt 14 durch die Zufuhröffnung 20 zugeführt. Auf Grund der besonderen oben beschriebenen Anordnung der Zuführöffnung 20, wird der sauerstoffhaltige Strom im Wesentlichen axial bzw. parallel bezüglich der Mittelachse 26 des Mischabschnitts 14 zugeführt. Der kohlenwasserstoffhaltige Strom durchdringt die Verteilerplatte 24 durch die Öffnungen 28 und wird so in den Mischabschnitt 14 gleichmäßig verteilt. In dem Mischabschnitt 14 vermischt sich der kohlenwasserstoffhaltige Strom intensiv mit dem sauerstoffhaltigen Strom, der durch die Zufuhröffnung 20 dem Mischabschnitt 14 zugeführt wird. Der zugeführte sauerstoffhaltige Strom wird dabei in dem Drallregister 22 mittels der Umlenkbleche verwirbelt bzw. verdrallt und sorgt so in dem Mischabschnitt 14 für eine bessere Vermischung mit dem kohlenwasserstoffhaltigen Strom. Das Drallregister 22 weist dabei eine Drallzahl von 0,3 bis 1, bevorzugt von 0,5 bis 0,7 und noch bevorzugter von 0,55 bis 0,65 aufweisen, beispielsweise 0,6. Mit anderen Worten sorgen die Umlenkbleche des Drallregisters 22dafür, dass der sauerstoffhaltige Strom einen Strömungszustand mit den genannten Drallzahlen aufweist.

Die Mischung aus dem kohlenwasserstoffhaltigen Strom und dem sauerstoffhaltigen Strom gelangt dann in das Mischrohr 30. Auf Grund des oben beschriebenen besonderen Verhältnisses von Länge und Durchmesser des Mischrohrs 30, weist die Mischung aus dem kohlenwasserstoffhaltigen Strom und dem sauerstoffhaltigen Strom in dem Mischrohr 30 eine Strömungsgeschwindigkeit von 0,2 Mach bis 1 Mach, bevorzugt von 0,3 Mach bis 0,9 Mach und noch bevorzugter von 0,4 Mach bis 0,8 Mach auf, beispielsweise 0,6 Mach. Diese hohe Strömungsgeschwindigkeit sorgt für eine weitere schnelle und effektive Verteilung bzw. Vermischung des kohlenwasserstoffhaltigen Stroms und des sauerstoffhaltigen Stroms.

Von dem Mischrohr 30 gelangt die Mischung aus dem kohlenwasserstoffhaltigen Strom und dem sauerstoffhaltigen Strom in den Mischdiffusor 16. In dem Mischdiffusor 16 erfährt die Mischung aus dem kohlenwasserstoffhaltigen Strom und dem sauerstoffhaltigen Strom auf Grund der oben beschriebenen Querschnittserweiterung des Mischdiffusors 16 in Strömungsrichtung gesehen eine Reduzierung der Strömungsgeschwindigkeit und eine Erhöhung des dynamischen Drucks. Von dem Mischdiffusor 16 gelangt die die Mischung aus dem kohlenwasserstoffhaltigen Strom und dem sauerstoffhaltigen Strom dann schließlich zu dem Brennerblock mit dem Feuerraum. In dem Feuerraum wird die Mischung aus dem kohlenwasserstoffhaltigen Strom und dem sauerstoffhaltigen Strom zum Herstellen von Acetylen zur Reaktion gebracht und anschließend durch eine Quencheinheit schnell abgekühlt.

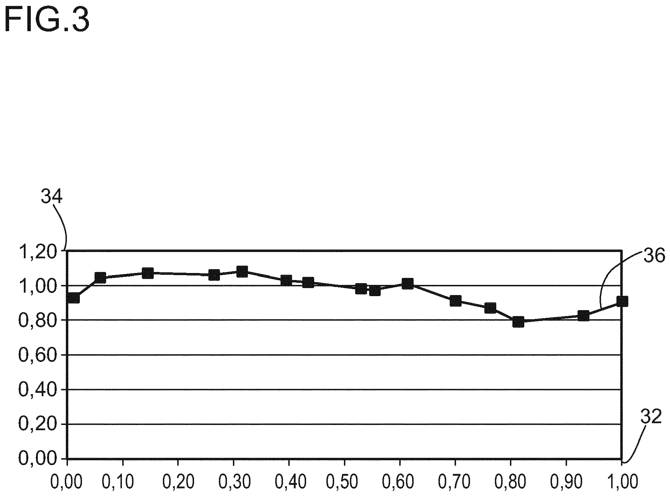

Figur 3 zeigt ein Simulationsergebnis einer Simulation einer lokalen Geschwindigkeit über den Umfang des Mischrohrs 30 im Verhältnis zu einer mittleren Geschwindigkeit. Auf der x-Achse 32 ist dabei die relative Position bezogen auf die Umfangsrichtung des Mischrohrs 30 angegeben, d. h. die Position X im Verhältnis zu π x Durchmesser des Mischrohrs 30. Auf der y-Achse 34 ist das Verhältnis der lokalen Geschwindigkeit zu der mittleren Geschwindigkeit als dimensionslose Kennzahl angegeben. Die Kurve 36 zeigt die bei der oben beschriebenen Vorrichtung 10 und dem erfindungsgemäßen Verfahren vorliegende lokale Geschwindigkeit über den Umfang des Mischrohrs 30 im Verhältnis zu einer mittleren Geschwindigkeit. Wie der Darstellung der Figur 3 anhand der Kurve 36 zu entnehmen ist, schwankt das Verhältnis der lokalen Geschwindigkeit zur mittleren Geschwindigkeit um den Wert 1,00 nur geringfügig. Dies bedeutet, dass eine gleichmäßige Geschwindigkeitsverteilung über den gesamten Umfang des Mischrohrs 30, d.h. die gesamte Querschnittsfläche des Mischrohrs 30, vorliegt. Entsprechend wird eine mögliche Fehlverteilung nach dem Drallregister 22 vermieden. Auch kann durch das Design des Drallregisters 22 und des Querschnitts der Mischeinheit 12 ein ungewollter hydrodynamischer Druckstoß in der Mischeinheit 12 vermieden werden. Dies kann durch die besondere Auslegung des Drallregisters 22 und der Verteilerplatte 24 erreicht werden.

### Bezugszeichenliste

- 10: Vorrichtung
- 12: Mischeinheit
- 14: Mischabschnitt
- 16: Mischdiffusor
- 18: Zufuhröffnung für Kohlenwasserstoffstrom
- 20: Zufuhröffnung für Sauerstoffstrom
- 22: Drallregister
- 24: Verteilerplatte
- 26: Mittelachse
- 28: Öffnungen
- 30: Mischrohr
- 32: X-Achse
- 34: Y-Achse
- 36: Kurve
- α: Halber Öffnungswinkel

## Patentansprüche

1. Vorrichtung (10) zum Herstellen von Acetylen und Synthesegas durch partielle Oxidation von Kohlenwasserstoffen mit Sauerstoff, umfassend eine Mischeinheit (12), einen Mischabschnitt und einen Mischdiffusor (16), wobei die Mischeinheit (12) eine Zufuhröffnung (18) zum Zuführen eines kohlenwasserstoffhaltigen Stroms, eine Zufuhröffnung (20) zum Zuführen eines sauerstoffhaltigen Stroms, ein Drallregister und eine Verteilerplatte (24) aufweist, wobei die Verteilerplatte (24) zwischen dem Mischabschnitt (14) und der Zufuhröffnung (18) zum Zuführen eines kohlenwasserstoffhaltigen Stroms angeordnet ist, wobei die Verteilerplatte (24) Öffnungen (28) aufweist, wobei das Drallregister (22) zwischen der Zufuhröffnung (20) zum Zuführen eines sauerstoffhaltigen Stroms und dem Mischabschnitt (14) angeordnet ist, wobei der Mischdiffusor (16) mit dem Mischabschnitt (14) verbunden ist.

2. Vorrichtung (10) nach dem vorhergehenden Anspruch, wobei der Mischabschnitt (14) im Wesentlichen zylindrisch ausgebildet ist, wobei die Verteilerplatte (24) so ausgebildet ist, dass die Verteilerplatte (24) den Mischabschnitt (14) in einer Umfangsrichtung gesehen vollständig umgibt.

3. Vorrichtung (10) nach einem der vorhergehenden Ansprüche, wobei das Drallregister (22) zumindest teilweise innerhalb der Verteilerplatte (24) angeordnet ist.

4. Vorrichtung (10) nach einem der vorhergehenden Ansprüche, wobei die Verteilerplatte (24) Öffnungen(28) zum Verteilen des kohlenwasserstoffhaltigen Stroms aufweist.

5. Vorrichtung (10) nach dem vorhergehenden Anspruch, wobei die Öffnungen(28) gleichmäßig oder ungleichmäßig verteilt in der Verteilerplatte (24) angeordnet sind.

6. Vorrichtung (10) nach dem vorhergehenden Anspruch, wobei die Verteilerplatte (24) so ausgebildet ist, dass das Verhältnis der Summe der Querschnittsflächen der Öffnungen(28) zu einer Oberfläche der Verteilerplatte (24) von 0,1 bis 0,4 ist.

7. Vorrichtung (10) nach einem der vorhergehenden Ansprüche, wobei die Zufuhröffnung (18) zum Zuführen eines kohlenwasserstoffhaltigen Stroms im Wesentlichen radial bezüglich einer Mittelachse (26) des Mischabschnitts (14) angeordnet ist.

8. Vorrichtung (10) nach einem der vorhergehenden Ansprüche, wobei die Zufuhröffnung zum Zuführen eines sauerstoffhaltigen Stroms im Wesentlichen parallel zu einer Mittelachse (26) des Mischabschnitts (14) angeordnet ist.

9. Vorrichtung (10) nach einem der vorhergehenden Ansprüche, wobei der Mischabschnitt (14) mit einem Mischrohr (30) verbunden ist, das eine Länge und einen Durchmesser aufweist, wobei die Länge und der Durchmesser so ausgebildet sind, dass eine Mischung aus dem sauerstoffhaltigen Strom und dem kohlenwasserstoffhaltigen Strom bei Betrieb der Vorrichtung (10) zum Herstellen von Acetylen und Synthesegas durch partielle Oxidation von Kohlenwasserstoffen mit Sauerstoff in dem Mischrohr (30) eine Strömungsgeschwindigkeit von 0,2 Mach bis 1 Mach aufweist.

10. Vorrichtung (10) nach einem der vorhergehenden Ansprüche, wobei das Drallregister (22) eine Drallzahl von 0,3 bis 1 aufweist.

11. Verfahren zum Herstellen von Acetylen und Synthesegas durch partielle Oxidation von Kohlenwasserstoffen mit Sauerstoff, umfassend eine Mischeinheit (12), einen Mischabschnitt (14) und einen Mischdiffusor (16), wobei ein kohlenwasserstoffhaltiger Strom zu dem Mischabschnitt (14) durch eine Zufuhröffnung (18) zugeführt wird, wobei ein sauerstoffhaltiger Strom zu dem Mischabschnitt (14) durch eine Zuführöffnung (20) zugeführt wird, wobei eine Verteilerplatte (24) zwischen dem Mischabschnitt (14) und der Zufuhröffnung (18) zum Zuführen eines kohlenwasserstoffhaltigen Stroms angeordnet wird, wobei die Verteilerplatte (24) Öffnungen(28) aufweist, wobei ein Drallregister (22) zwischen der Zufuhröffnung (20) zum Zuführen eines sauerstoffhaltigen Stroms und dem Mischabschnitt (14) angeordnet wird, wobei ein Mischdiffusor (16) mit dem Mischabschnitt (14) verbunden wird.

12. Verfahren nach dem vorhergehenden Anspruch, wobei die Verteilerplatte (24) so ausgebildet ist, dass das Verhältnis der Summe der Querschnittsflächen der Öffnungen(28) zu einer Oberfläche der Verteilerplatte (24) von 0,1 bis 0,4 ist.

13. Verfahren nach einem der beiden vorhergehenden Ansprüche, wobei der kohlenwasserstoffhaltige Strom im Wesentlichen radial bezüglich einer Mittelachse (26) des Mischabschnitts (14) zugeführt wird, wobei der sauerstoffhaltige Strom im Wesentlichen parallel zu einer Mittelachse (26) des Mischabschnitts (14) zugeführt wird.

14. Verfahren nach einem der drei vorhergehenden Ansprüche, wobei das Mischrohr (30) eine Länge und einen Durchmesser aufweist, wobei die Länge und der Durchmesser so ausgebildet sind, dass das Verfahren zum Herstellen von Acetylen und Synthesegas durch partielle Oxidation von Kohlenwasserstoffen mit Sauerstoff so durchgeführt wird, dass eine Mischung aus dem sauerstoffhaltigen Strom und dem kohlenwasserstoffhaltigen Strom in dem Mischrohr (30) eine Strömungsgeschwindigkeit von 0,2 Mach bis 1 Mach aufweist.

15. Verfahren nach einem der vier vorhergehenden Ansprüche, wobei das Drallregister (22) eine Drallzahl von 0,3 bis 1 aufweist.

## Claims

1. An apparatus (10) for preparing acetylene and synthesis gas by partial oxidation of hydrocarbons with oxygen, comprising a mixing unit (12), a mixing section and a mixing diffuser (16), wherein the mixing unit (12) has a feed orifice (18) for supply of a hydrocarbonaceous stream, a feed orifice (20) for supply of an oxygenous stream, a swirl register and a distributor plate (24), wherein the distributor plate (24) is disposed between the mixing section (14) and the feed orifice (18) for supply of a hydrocarbonaceous stream, wherein the distributor plate (24) has orifices (28), wherein the swirl register (22) is disposed between the feed orifice (20) for supply of an oxygenous stream and the mixing section (14), wherein the mixing diffuser (16) is connected to the mixing section (14).

2. The apparatus (10) according to the preceding claim, wherein the mixing section (14) is essentially cylindrical, wherein the distributor plate (24) is configured such that the distributor plate (24) completely surrounds the mixing section (14), viewed in a circumferential direction.

3. The apparatus (10) according to either of the preceding claims, wherein the swirl register (22) is disposed at least partly within the distributor plate (24).

4. The apparatus (10) according to any of the preceding claims, wherein the distributor plate (24) has orifices (28) for distribution of the hydrocarbonaceous stream.

5. The apparatus (10) according to the preceding claim, wherein the orifices (28) are arranged in homogeneous or inhomogeneous distribution in the distributor plate (24).

6. The apparatus (10) according to the preceding claim, wherein the distributor plate (24) is configured such that the ratio of the sum total of the cross-sectional areas of the orifices (28) to a surface area of the distributor plate (24) is from 0.1 to 0.4.

7. The apparatus (10) according to any of the preceding claims, wherein the feed orifice (18) for supply of a hydrocarbonaceous stream is arranged essentially radially with respect to a center axis (26) of the mixing section (14).

8. The apparatus (10) according to any of the preceding claims, wherein the feed orifice for supply of an oxygenous stream is arranged essentially parallel to a center axis (26) of the mixing section (14).

9. The apparatus (10) according to any of the preceding claims, wherein the mixing section (14) is connected to a mixing tube (30) having a length and a diameter, wherein the length and diameter are configured such that a mixture of the oxygenous stream and the hydrocarbonaceous stream in operation of the apparatus (10) for preparing acetylene and synthesis gas by partial oxidation of hydrocarbons with oxygen in the mixing tube (30) has a flow velocity of 0.2 mach to 1 mach.

10. The apparatus (10) according to any of the preceding claims, wherein the swirl register (22) has a swirl number of 0.3 to 1.

11. A process for preparing acetylene and synthesis gas by partial oxidation of hydrocarbons with oxygen, comprising a mixing unit (12), a mixing section (14) and a mixing diffuser (16), wherein a hydrocarbonaceous stream is fed to the mixing section (14) through a feed orifice (18), wherein an oxygenous stream is fed to the mixing section (14) through a feed orifice (20), wherein a distributor plate (24) is arranged between the mixing section (14) and the feed orifice (18) for supply of a hydrocarbonaceous stream, wherein the distributor plate (24) has orifices (28), wherein a swirl register (22) is disposed between the feed orifice (20) for supply of an oxygenous stream and the mixing section (14), wherein a mixing diffuser (16) is connected to the mixing section (14).

12. The process according to the preceding claim, wherein the distributor plate (24) is configured such that the ratio of the sum total of the cross-sectional areas of the orifices (28) to a surface area of the distributor plate (24) is from 0.1 to 0.4.

13. The process according to either of the two preceding claims, wherein the hydrocarbonaceous stream is fed in essentially radially with respect to a center axis (26) of the mixing section (14), wherein the oxygenous stream is fed in essentially parallel to a center axis (26) of the mixing section (14).

14. The process according to any of the three preceding claims, wherein the mixing tube (30) has a length and a diameter, the length and diameter being configured such that the process for preparing acetylene and synthesis gas by partial oxidation of hydrocarbons with oxygen is conducted such that a mixture of the oxygenous stream and the hydrocarbonaceous stream in the mixing tube (30) has a flow velocity of 0.2 mach to 1 mach.

15. The process according to any of the four preceding claims, wherein the swirl register (22) has a swirl number of 0.3 to 1.

## Revendications

1. Dispositif (10) pour la fabrication d'acétylène et de gaz de synthèse par oxydation partielle d'hydrocarbures avec de l'oxygène, comprenant une unité de mélange (12), une section de mélange et un diffuseur de mélange (16), l'unité de mélange (12) comprenant une ouverture d'alimentation (18) pour l'alimentation d'un courant contenant des hydrocarbures, une ouverture d'alimentation (20) pour l'alimentation d'un courant contenant de l'oxygène, un registre de rotation et une plaque de distribution (24), la plaque de distribution (24) étant agencée entre la section de mélange (14) et l'ouverture d'alimentation (18) pour l'alimentation d'un courant contenant des hydrocarbures, la plaque de distribution (24) comprenant des ouvertures (28), le registre de rotation (22) étant agencé entre l'ouverture d'alimentation (20) pour l'alimentation d'un courant contenant de l'oxygène et la section de mélange (14), le diffuseur de mélange (16) étant raccordé avec la section de mélange (14).

2. Dispositif (10) selon la revendication précédente, dans lequel la section de mélange (14) est configurée sous forme essentiellement cylindrique, la plaque de distribution (24) étant configurée de sorte que la plaque de distribution (24) entoure entièrement la section de mélange (14) dans une direction périphérique.

3. Dispositif (10) selon l'une quelconque des revendications précédentes, dans lequel le registre de rotation (22) est agencé au moins en partie dans la plaque de distribution (24).

4. Dispositif (10) selon l'une quelconque des revendications précédentes, dans lequel la plaque de distribution (24) comprend des ouvertures (28) pour la distribution du courant contenant des hydrocarbures.

5. Dispositif (10) selon la revendication précédente, dans lequel les ouvertures (28) sont réparties de manière uniforme ou non uniforme dans la plaque de distribution (24).

6. Dispositif (10) selon la revendication précédente, dans lequel la plaque de distribution (24) est configurée de sorte que le rapport entre la somme des surfaces de section transversale des ouvertures (28) et une surface de la plaque de distribution (24) soit de 0,1 à 0,4.

7. Dispositif (10) selon l'une quelconque des revendications précédentes, dans lequel l'ouverture d'alimentation (18) pour l'alimentation d'un courant contenant des hydrocarbures est agencée essentiellement radialement par rapport à un axe central (26) de la section de mélange (14).

8. Dispositif (10) selon l'une quelconque des revendications précédentes, dans lequel l'ouverture d'alimentation pour l'alimentation d'un courant contenant de l'oxygène est agencée essentiellement parallèlement à un axe central (26) de la section de mélange (14).

9. Dispositif (10) selon l'une quelconque des revendications précédentes, dans lequel la section de mélange (14) est raccordée à un tube de mélange (30), qui présente une longueur et un diamètre, la longueur et le diamètre étant configurés de sorte qu'un mélange du courant contenant de l'oxygène et du courant contenant des hydrocarbures présente lors de l'exploitation du dispositif (10) pour la fabrication d'acétylène et de gaz de synthèse par oxydation partielle d'hydrocarbures avec de l'oxygène dans le tube de mélange (30) une vitesse d'écoulement de 0,2 Mach à 1 Mach.

10. Dispositif (10) selon l'une quelconque des revendications précédentes, dans lequel le registre de rotation (22) présente un nombre de rotations de 0,3 à 1.

11. Procédé de fabrication d'acétylène et de gaz de synthèse par oxydation partielle d'hydrocarbures avec de l'oxygène, comprenant une unité de mélange (12), une section de mélange (14) et un diffuseur de mélange (16), un courant contenant des hydrocarbures étant introduit dans la section de mélange (14) par une ouverture d'alimentation (18), un courant contenant de l'oxygène étant introduit dans la section de mélange (14) par une ouverture d'alimentation (20), une plaque de distribution (24) étant agencée entre la section de mélange (14) et l'ouverture d'alimentation (18) pour l'alimentation d'un courant contenant des hydrocarbures, la plaque de distribution (24) comprenant des ouvertures (28), un registre de rotation (22) étant agencé entre l'ouverture d'alimentation (20) pour l'alimentation d'un courant contenant de l'oxygène et la section de mélange (14), un diffuseur de mélange (16) étant raccordé avec la section de mélange (14).

12. Procédé selon la revendication précédente, dans lequel la plaque de distribution (24) est configurée de sorte que le rapport entre la somme des surfaces de section transversale des ouvertures (28) et une surface de la plaque de distribution (24) soit de 0,1 à 0,4.

13. Procédé selon l'une quelconque des deux revendications précédentes, dans lequel le courant contenant des hydrocarbures est introduit essentiellement radialement par rapport à un axe central (26) de la section de mélange (14), le courant contenant de l'oxygène étant introduit essentiellement parallèlement à un axe central (26) de la section de mélange (14).

14. Procédé selon l'une quelconque des trois revendications précédentes, dans lequel le tube de mélange (30) présente une longueur et un diamètre, la longueur et le diamètre étant configurés de sorte que le procédé de fabrication d'acétylène et de gaz de synthèse par oxydation partielle d'hydrocarbures avec de l'oxygène soit réalisé de sorte qu'un mélange du courant contenant de l'oxygène et du courant contenant des hydrocarbures dans le tube de mélange (30) présente une vitesse d'écoulement de 0,2 Mach à 1 Mach.

15. Procédé selon l'une quelconque des quatre revendications précédentes, dans lequel le registre de rotation (22) présente un nombre de rotations de 0,3 à 1.
